Europäisches Patentamt

European Patent Office

Office européen des brevets

(11) Veröffentlichungsnummer: **0 341 199**

**A1**

(12) # EUROPÄISCHE PATENTANMELDUNG

(21) Anmeldenummer: **89810216.5**

(22) Anmeldetag: **20.03.89**

(51) Int. Cl.⁴: **A 61 F 2/34**

(30) Priorität: **25.04.88 CH 1534/88**

(43) Veröffentlichungstag der Anmeldung:
**08.11.89 Patentblatt 89/45**

(84) Benannte Vertragsstaaten: **AT DE FR GB IT**

(71) Anmelder: **GEBRÜDER SULZER AKTIENGESELLSCHAFT**
**Zürcherstrasse 9**
**CH-8401 Winterthur (CH)**

**Protek AG**
**Stadtbachstrasse 64**
**CH-3001 Bern (CH)**

(72) Erfinder: **Wagner, Heinz, Prof. Dr.-med. Orthopädische Klinik Wichernhaus Krankenhaus Rummelsberg D-8501 Schwarzenbruck (DE)**

(54) **Künstliche Hüftgelenkspfanne.**

(57) Ueber den Bereich der Aussenschale (1), in dem Durchtrittsöffnungen (6) vorhanden sind, ist der Kunststoff-Pfannenkörper (11) mit einer metallischen Trennschicht (15) belegt.

Durch diese Trennschicht (15) wird ein Kaltfliessen des Kunststoffes des Pfannenkörpers (11) in die Durchtrittsöffnungen (6) hinein verhindert; zusätzlich werden aus medizinischen Gründen unerwünschte, grossflächige direkte Kontakte zwischen Knochengewebe und Kunststoff vermieden.

Fig.1

EP 0 341 199 A1

**Beschreibung**

### Künstliche Hüftgelenkspfanne

Die Erfindung betrifft eine künstliche Hüftgelenkspfanne, bestehend aus einer metallenen Aussenschale und aus einem, die Pfannenschale für die Aufnahme eines Gelenkkopfes enthaltenden Kunststoff-Pfannenkörper, der in der Aussenschale verankerbar ist, wobei die Aussenschale mit einer Vielzahl von Durchtrittsöffnungen für Knochenschrauben versehen ist.

Eine Hüftgelenkspfanne der vorstehend genannten Art ist bekannt aus der CH-Anmeldung 1219/87-0; die Vielzahl der Durchtrittsöffnungen für Knochenschrauben in der Aussenschale sollen dem Operateur eine Anzahl von Auswahlmöglichkeiten für das Setzen und Verankern der Schrauben im Knochen bieten. Daher werden von diesen Oeffnungen im individuellen Fall im allgemeinen nur eine oder wenige benutzt.

In der Praxis hat sich nun gezeigt, dass unter den Beanspruchungen der implantierten Pfanne der Kunststoff-Pfannenkörper kaltfliesst und in die "freibleibenden" Durchtrittsöffnungen eindringt. Das führt zu Deformationen des Pfannenkörpers und der Pfannenschale, wodurch in dieser erhöhter Abrieb entsteht.

Aufgabe der Erfindung ist es, den Kaltfluss des Pfannenkörpers in die freien Durchtrittsöffnungen zu verhindern; erreicht wird dieses Ziel dadurch, dass die äussere Oberfläche des Pfannenkörpers mindestens im Bereich der Durchtrittsöffnungen der Aussenschale mit einer metallischen Trennschicht belegt ist.

Die Abdeckung der freien Durchtrittsöffnungen bewirkt dabei nicht nur, dass ein Kaltfliessen des Pfannenkörpers in die Durchtrittsöffnungen hinein unterdrückt wird, sondern verhindert auch direkte Kontakte zwischen Kunststoff und lebendem Gewebe im Bereich der Oeffnungen mit Sicherheit.

Eine vorteilhafte Konstruktion für die Trennschicht ergibt sich, wenn die Trennschicht in einer in Umfangsrichtung geschlossenen Einlage in den Pfannenkörper besteht; denn in diesem Fall kann der Pfannenkörper in jeder beliebigen Umfangs-Winkellage relativ zur Aussenschale eingesetzt werden, ohne dass auf einer Deckung der Trennschicht mit den Durchtrittsöffnungen geachtet werden muss. Dies ist besonders für Pfannenkörper von Bedeutung, die in Umfangsrichtung nicht rotationssymmetrisch sind, also beispielsweise eine einseitige Pfannendacherhöhung haben.

Eine Fixierung der Trennschicht, die aus einem mehrlagigen Drahtgitter oder aus einer geschlossenen Platte bestehen kann, auf dem Pfannenkörper lässt sich auf einfache Weise durch Schnappverschlüsse bewerkstelligen, die am äquatorialen bzw. polseitigen Rand der Trennschicht vorgesehen sind. Ebenso besteht die Verbindung zwischen Pfannenkörper und Aussenschale in bekannter Weise in einer konischen Hinterschneidung der Aussenschale und einem dazu passenden Konus am Pfannenkörper.

Im folgenden wird die Erfindung anhand eines Ausführungsbeispiels im Zusammenhang mit der Zeichnung erläutert.

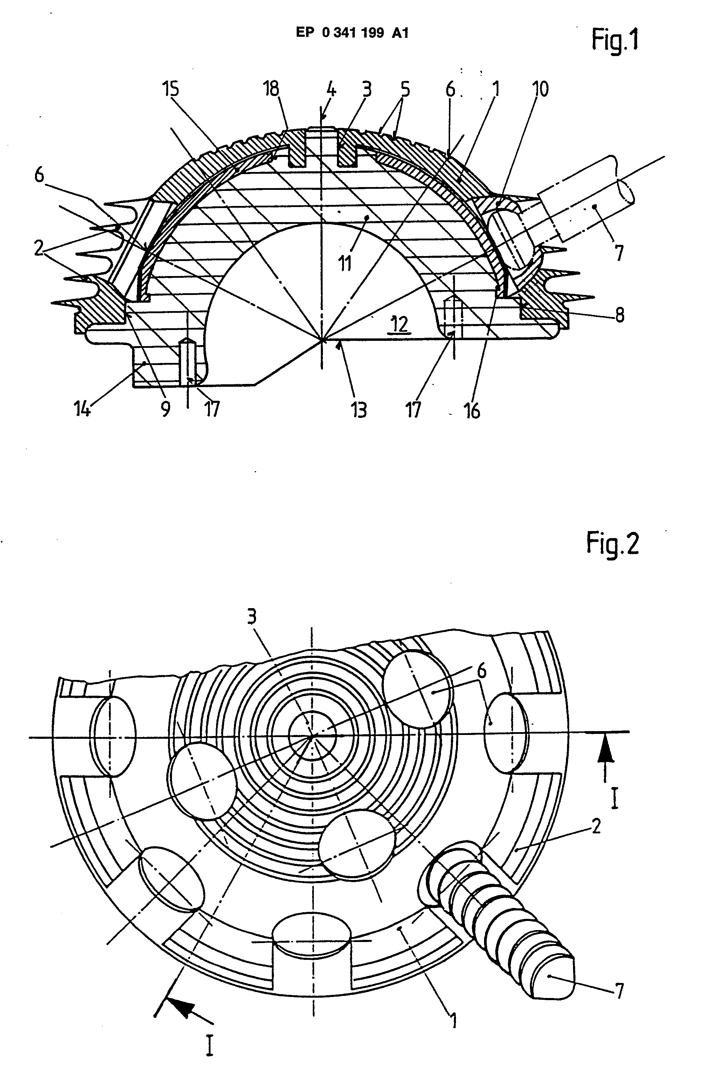

Fig. 1 zeigt einen Schnitt I-I von Fig. 2 durch die Pfannenkonstruktion;

Fig. 2 gibt eine Aufsicht in Richtung der Polachse auf die Aussenschale wieder.

Die beispielsweise aus Titan bestehende Aussenschale 1 ist im Bereich niedriger "geographischer" Breiten mit einem Aussengewinde 2 versehen, mit dem sie in den Beckenknochen eingeschraubt wird. An ihrem Pol, durch den die gleichzeitig eine Rotationssymmetrieachse der Aussenschale bildende Polachse 4 verläuft, weist sie eine Bohrung 3 für den Einsatz eines nicht gezeigten Setzinstrumentes auf. Kreisringförmige Rillen 5 dienen in der Umgebung des Poles dem Ein- und Anwachsen von Gewebe. Mit ihren Zentren auf zwei verschiedenen "Breiten" sind in der Aussenschale 1 über den Umfang verteilt Durchtrittsbohrungen 6 für Knochenschrauben 7 vorgesehen. Diese Bohrungen 6 sind auf der Innenseite der Schale 1 so erweitert, dass in sie eine Konushülse 10 eingelegt werden kann, in der der nur angedeutete Schraubenkopf, in der Bohrung 6 versenkt, gelagert wird. Die Konushülse 10 vergrössert die Variationsmöglichkeiten für die Einschraubrichtungen der Schraube 7.

Am äquatorialen Rand ist der Innenhohlraum der Aussenschale 1 zur Aufnahme eines Kunststoff-Pfannenkörpers 11 zweimal stufenförmig abgesetzt. Der durchmesserkleinere "innere" Absatz 8 ist dabei als sich nach aussen leicht erweiternder Konus ausgebildet, in den zur Verbindung von Schale 1 und Körper 11 ein entsprechender Konus 9 des Pfannenkörpers 11 einschnappt.

Der in der Aussenschale 1 gelagerte Pfannenkörper 11 enthält die eigentliche Pfannenschale 12 für die Aufnahme eines nicht gezeigten Gelenkkopfes. Bei der dargestellten Ausführungsform ist der Pfannenkörper 11 auf einem Teil seines Umfangs über die Aequatorebene 13 hinaus verlängert. Die Verlängerung 14 weist bei der implantierten Pfanne nach lateral und hat die Aufgabe, Luxationen des Gelenkkopfes beim extremen Abbiegen des Hüftgelenkes zu vermeiden.

In der kugeligen, äusseren Oberfläche des Pfannenkörpers 11 ist als Trennschicht ein aus einem Titan-Blech gefertigter Schalenkörper 15 eingelegt, der sich über den Bereich geographischer Breiten erstreckt, in denen die Durchtrittsöffnungen 6 der Aussenschale 1 liegen. Der Schalenkörper 15 wird im Kunststoff durch über den ganzen Umfang verlaufende Schnappverschlüsse 16 und 18 an seinem äquatorialen bzw. polseitigen Rand gehalten.

Für den Eingriff eines Setzinstrumentes sind im Rand des Pfannenkörper 11 auf dem Umfang schliesslich Bohrungen 17 verteilt.

Eine Implantation der neuen Hüftgelenkspfanne kann beispielsweise derart erfolgen, dass zunächst die Aussenschale mit ihrem Gewinde 2 durch ein in mindestens einige der Bohrungen 6 eingreifendes, in der Bohrung 3 zentriertes Einschraubinstrument in den vorbereiteten Beckenknochen eingeschraubt wird. Anschliessend wird die Aussenschale 1 gegebenenfalls zusätzlich mit Hilfe von Knochenschrauben 7 verankert. Mit einem Setzinstrument, das in die Bohrungen 17 eingreift, wird der Pfannenkörper 11 in der richtigen Lage der Verlängerung 14 dann in die Aussenchale 1 eingepresst, wobei die beiden Konen 8 und 9 ineinander einschnappen.

**Patentansprüche**

1. Künstliche Hüftgelenkspfanne, bestehend aus einer metallenen Aussenschale und aus einem, die Pfannenschale für die Aufnahme eines Gelenkkopfes enthaltenden Kunststoff-Pfannenkörper, der in der Aussenschale verankerbar ist, wobei die Aussenschale mit einer Vielzahl von Durchtrittsöffnungen für Knochenschrauben versehen ist, **dadurch gekennzeichnet, dass** die äussere Oberfläche des Pfannenkörpers (11) mindestens im Bereich der Durchtrittsöffnungen (6) der Aussenschale (1) mit einer metallischen Trennschicht (15) belegt ist.

2. Hüftgelenkspfanne nach Anspruch 1, **dadurch gekennzeichnet, dass** die Trennschicht (15) in einer in Umfangsrichtung geschlossenen Einlage in den Pfannenkörper (11) besteht.

3. Hüftgelenkspfanne nach Anspruch 2, **dadurch gekennzeichnet, dass** die Trennschicht (15) durch je einen Schnappverschluss (16,18) an ihrem äquator- und ihrem polseitigen Rand auf dem Pfannenkörper (11) gehalten ist.

EP 0 341 199 A1

Fig.1

Fig.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A-2 597 329 (S. CUILLERON) * Figur 8; Seite 5, Zeilen 13-30 * | 1,2 | A 61 F 2/34 |
| Y | | 3 | |
| | --- | | |
| Y | FR-A-2 597 747 (LA GRANGE et al.) * Figur 2 * | 3 | |
| | --- | | |
| A | DE-C-3 535 959 (ORTHOPLANT ENDOPROTHETIC GmbH) * Figur 1; Spalte 3, Zeilen 16-19 * | 1-3 | |
| | ----- | | |

| RECHERCHIERTE SACHGEBIETE (Int. Cl.4) |
|---|
| A 61 F |

Der vorliegende Recherchenbericht wurde für alle Patentansprüche erstellt

| Recherchenort | Abschlußdatum der Recherche | Prüfer |
|---|---|---|
| DEN HAAG | 20-07-1989 | ARGENTINI A. |